# EUROPEAN PATENT APPLICATION

(11) **EP 2 610 635 A2**
(43) Date of publication of application: **03.07.2013**
(21) Application number: 12199548.4
(22) Date of filing: 27.12.2012
(51) Int. Cl.: G01S 7/52, G01S 15/89

(54) **Providing Doppler spectrum images corresponding to at least two sample volumes in ultrasound system**

(30) Priority: 29.12.2011 KR 20110145659
(71) Applicant: Samsung Medison Co., Ltd., Gangwon-do 250-870 (KR)
(72) Inventor: Lee, Han Woo, 135-851 Seoul (KR); Kim, Hyoung Jin, 135-851 Seoul (KR)
(74) Representative: Lorenz, Markus

(57) **Abstract**

There are provided embodiments for providing Doppler spectrum images corresponding to at least two sample volumes. In one embodiment, by way of nonlimiting example, an ultrasound system comprises: a processing unit configured to form at least two Doppler spectrum images corresponding to at least two sample volumes based on ultrasound data corresponding to the at least two sample volume, the processing unit being further configured to perform an image process for connecting the at least two sample volumes to the at least two Doppler spectrum images.

## Description

### CROSS-REFERENCE TO RELATED APPLICATIONS

The present application claims priority from Korean Patent Application No. 10-2011-0145659 filed on December 29, 2011.

### TECHNICAL FIELD

The present disclosure generally relates to ultrasound systems, and more particularly to providing Doppler spectrum images corresponding to at least two sample volumes in an ultrasound system.

### BACKGROUND

An ultrasound system has become an important and popular diagnostic tool since it has a wide range of applications. Specifically, due to its non-invasive and non-destructive nature, the ultrasound system has been extensively used in the medical profession. Modem high-performance ultrasound systems and techniques are commonly used to produce two-dimensional or three-dimensional ultrasound images of internal features of target objects (e.g., human organs).

The ultrasound system provides ultrasound images of various modes including a brightness mode image representing reflection coefficients of ultrasound signals (i.e., ultrasound echo signals) reflected from a target object of a living body with a two-dimensional image, a Doppler mode image representing velocity of a moving target object with spectral Doppler by using a Doppler effect, a color Doppler mode image representing velocity of the moving target object with colors by using the Doppler effec\t, an elastic image representing mechanical characteristics of tissues before and after applying compression thereto, and the like.

The ultrasound system transmits the ultrasound signals to the living body and receives the ultrasound echo signals from the living body to form Doppler signals corresponding to a region of interest, which is set on the brightness mode image. The ultrasound system further forms the color Doppler mode image representing the velocity of the moving target object with colors based on the Doppler signals. In particular, the color Doppler image represents the motion of the target object (e.g., blood flow) with the colors. The color Doppler image is used to diagnose disease of a blood vessel, a heart and the like. However, it is difficult to represent an accurate motion of the target object (e.g., blood flow) since the respective colors indicated by a motion value is a function of the velocity of the target object, which moves forward in a transmission direction of the ultrasound signals and moves backward in the transmission direction of the ultrasound signals.

Particularly, the ultrasound system sets a sample volume on the brightness mode image, transmits ultrasound signals to the living body based on an ensemble number, and receives ultrasound echo signals from the living to form a Doppler spectrum image corresponding to the sample volume.

### SUMMARY

There are provided embodiments for forming at least two Doppler spectrum images corresponding to at least two sample volumes, and performing an image process for connecting the at least two Doppler spectrum images to the at least two sample volumes.

In one embodiment, by way of non-limiting example, an ultrasound system comprises: a processing unit configured to form at least two Doppler spectrum images corresponding to at least two sample volumes based on ultrasound data corresponding to the at least two sample volume, the processing unit being further configured to perform an image process for connecting the at least two sample volumes to the at least two Doppler spectrum images.

In another embodiment, there is provided a method of providing Doppler spectrum images, comprising: a) forming at least two Doppler spectrum images corresponding to at least two sample volumes based on ultrasound data corresponding to the at least two sample volumes; and b) performing an image process for connecting the at least two sample volumes to the at least two Doppler spectrum images.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

FIG. 1 is a block diagram showing an illustrative embodiment of an ultrasound system.
FIG. 2 is a schematic diagram showing an example of a brightness mode image and sample volumes.
FIG. 3 is a block diagram showing an illustrative embodiment of an ultrasound data acquiring unit.
FIG. 4 is a schematic diagram showing an example of sampling data and pixels of an ultrasound image.
FIGS. 5 to 8 are schematic diagrams showing examples of performing a reception beam-forming.
FIG. 9 is a schematic diagram showing an example of setting weights.
FIG. 10 is a schematic diagram showing an example of setting a sampling data set.
FIG. 11 is a flow chart showing a process of forming Doppler spectrum images.
FIGS. 12 and 13 are schematic diagrams showing examples of first connection information and second connection information.

### DETAILED DESCRIPTION

A detailed description may be provided with reference to the accompanying drawings. One of ordinary skill in the art may realize that the following description is illustrative only and is not in any way limiting.

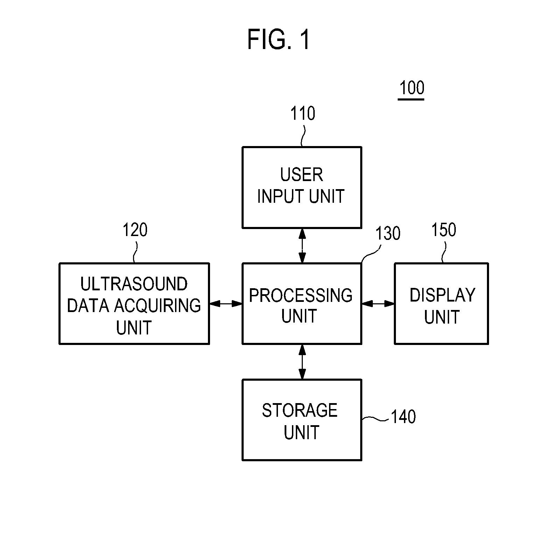

Referring to FIG. 1, an ultrasound system 100 in accordance with an illustrative embodiment is shown. As depicted therein, the ultrasound system 100 includes a user input unit 110.

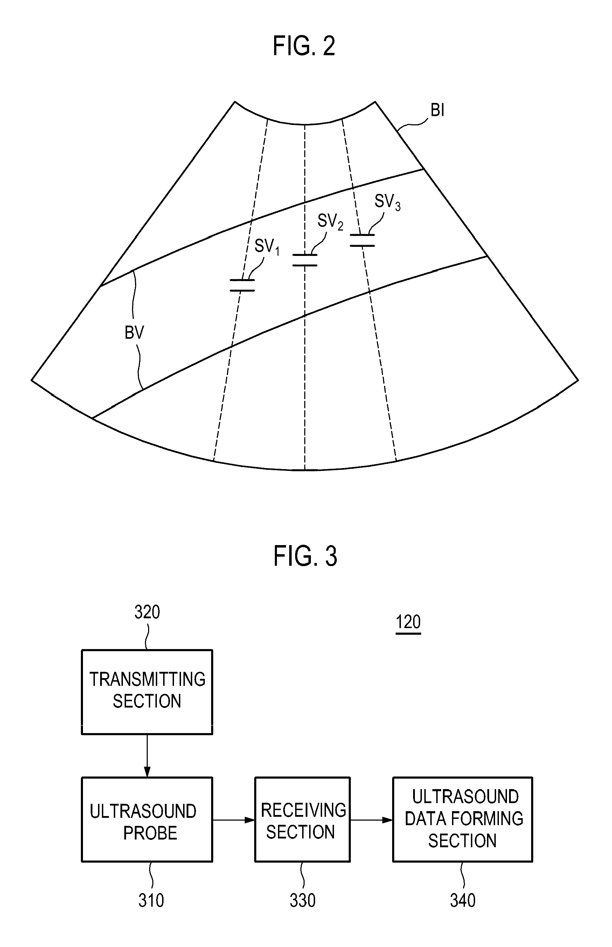

The user input unit 110 is configured to receive input information from a user. In one embodiment, the input information includes information for setting at least two sample volumes (e.g., SV₁, SV₂, SV₃) on a brightness mode image BI, as shown in FIG. 2. However, it should be noted herein that the input information may not be limited thereto. In FIG. 2, the reference numeral BV represents a blood vessel. The user input unit 110 includes a control panel, a track ball, a touch screen, a mouse, a keyboard and the like.

The ultrasound system 100 further includes an ultrasound data acquiring unit 120. The ultrasound data acquiring unit 120 is configured to transmit ultrasound signals to a living body. The living body includes target objects (e.g., blood vessel, heart, blood flow, etc). The ultrasound data acquiring unit 120 is further configured to receive ultrasound signals (i.e., ultrasound echo signals) from the living body to acquire ultrasound data corresponding to an ultrasound image.

FIG. 3 is a block diagram showing an illustrative embodiment of the ultrasound data acquiring unit. Referring to FIG. 3, the ultrasound data acquiring unit 120 includes an ultrasound probe 310.

The ultrasound probe 310 includes a plurality of elements (not shown) for reciprocally converting between ultrasound signals and electrical signals. The ultrasound probe 310 is configured to transmit the ultrasound signals to the living body. The ultrasound signals transmitted from the ultrasound probe 310 are non-focused ultrasound signals or focused ultrasound signals. That is, the ultrasound signals (i.e., transmissions beam) include general transmission signals (i.e., transmission beam) that a focal point locates at the inside of an imaging region, broad transmission signals that the focal point locates at the outside of the imaging region, plane wave transmission signals that the focal point locates at infinity, virtual apex transmission signals that the focal point locates at the back of surface of the ultrasound probe 310 and the like. The ultrasound probe 310 is further configured to receive the ultrasound echo signals from the living body to output electrical signals (hereinafter referred to as "reception signals"). The reception signals are analog signals. The ultrasound probe 310 includes a convex probe, a linear probe and the like.

The ultrasound data acquiring unit 120 further includes a transmitting section 320. The transmitting section 320 is configured to control the transmission of the ultrasound signals. The transmitting section 320 is also configured to generate electrical signals (hereinafter referred to as "transmission signals") in consideration of the elements.

In one embodiment, the transmitting section 320 is configured to generate transmission signals (hereinafter referred to as "brightness mode transmission signals") for obtaining the brightness mode image BI in consideration of the elements. Thus, the ultrasound probe 310 is configured to convert the brightness mode transmission signals provided from the transmitting section 320 into the ultrasound signals, transmit the ultrasound signals to the living body, and receive the ultrasound echo signals from the living body to output reception signals (hereinafter referred to as "brightness mode reception signals").

The transmitting section 320 is further configured to generate transmission signals (hereinafter referred to as "Doppler mode transmission signals") for obtaining Doppler spectrum images corresponding to the at least two sample volumes based on an ensemble number. The ensemble number represents the number of transmitting and receiving the ultrasound signals. Thus, the ultrasound probe 310 is configured to convert the Doppler mode transmission signals provided from the transmitting section 320 into the ultrasound signals, transmit the ultrasound signals to the living body, and receive the ultrasound echo signals from the living body in at least one reception direction to output reception signals (hereinafter referred to as "Doppler mode reception signals"). The ultrasound signals transmitted from the ultrasound probe 310 are the plane wave signals.

The ultrasound data acquiring unit 120 further includes a receiving section 330. The receiving section 330 is configured to perform an analog-digital conversion upon the reception signals provided from the ultrasound probe 310 to form sampling data. The receiving section 330 is additionally configured to perform a reception beam-forming upon the sampling data in consideration of the elements to form reception-focused data. The reception beam-forming will be described below in detail.

In one embodiment, the receiving section 330 is configured to perform the analog-digital conversion upon the brightness mode reception signals provided from the ultrasound probe 310 to form sampling data (hereinafter referred to as "brightness mode sampling data"). The receiving section 330 is further configured to perform the reception beam-forming upon the brightness mode sampling data to form reception-focused data (hereinafter referred to as "brightness mode reception-focused data").

The receiving section 330 is further configured to perform the analog-digital conversion upon the Doppler mode reception signals provided from the ultrasound probe 310 to form sampling data (hereinafter referred to as "Doppler mode sampling data"). The receiving section 330 is further configured to perform the reception beam-forming upon the Doppler mode sampling data to form reception-focused data (hereinafter referred to as "Doppler mode reception-focused data") corresponding to the at least two sample volumes.

For example, the receiving section 330 performs the reception beam-forming upon the Doppler mode sampling data to form first Doppler mode reception-focused data corresponding to the sample volume SV₁. The receiving section 330 further performs the reception beam-forming upon the Doppler mode sampling data to form second Doppler mode reception-focused data corresponding to the sample volume SV₂. The receiving section 330 further performs the reception beam-forming upon the Doppler mode sampling data to form third Doppler mode reception-focused data corresponding to the sample volume SV₃.

The reception beam-forming is described with reference to the accompanying drawings.

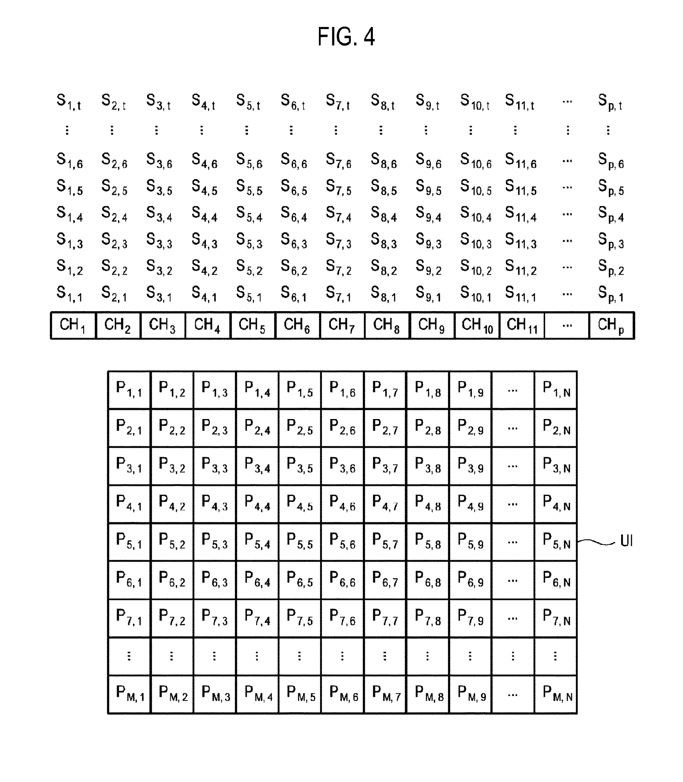

In one embodiment, the receiving section 330 is configured to perform the analog-digital conversion upon the reception signals provided through a plurality of channels CHₖ, wherein 1≤k≤N, from the ultrasound probe 310 to form sampling data S_{i,j}, wherein the i and j are a positive integer, as shown in FIG. 4. The sampling data S_{i,j} are stored in a storage unit 140. The receiving section 330 is further configured to detect pixels corresponding to the sampling data based on positions of the elements and positions (orientation) of pixels of the ultrasound image UI with respect to the elements. That is, the receiving section 330 selects the pixels, which the respective sampling data are used as pixel data thereof, during the reception beam-forming based on the positions of the elements and the orientation of the respective pixels of the ultrasound image UI with respect to the elements. The receiving section 330 is configured to cumulatively assign the sampling data corresponding to the selected pixels as the pixel data.

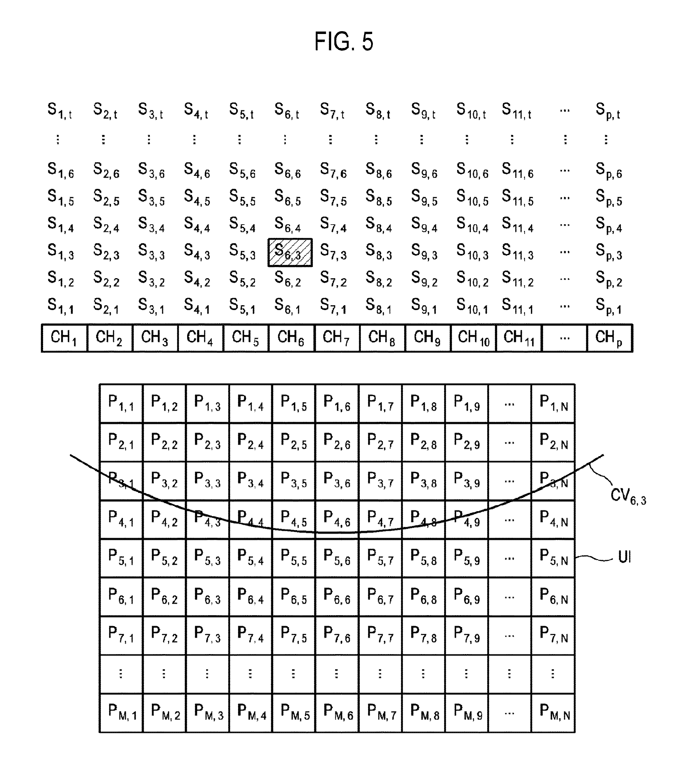

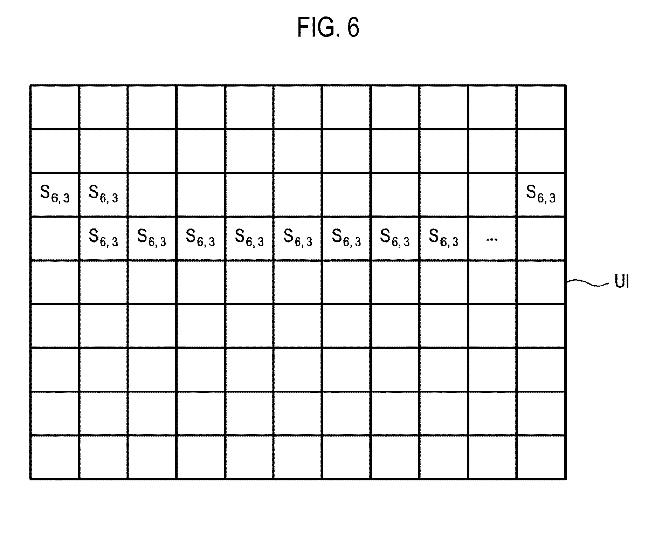

For example, the receiving section 330 is configured to set a curve (hereinafter referred to as "reception beam-forming curve") CV₆,₃ for selecting pixels, which the sampling data S_{6,3} are used as the pixel data thereof, during the reception beam-forming based on the positions of the elements and the orientation of the respective pixels of the ultrasound image UI with respect to the elements, as shown in FIG. 5. The receiving section 330 is further configured to detect the pixels P_{3,1}, P_{3,2}, P_{4,2}, P_{4,3}, P_{4,4}, P_{4,5}, P_{4,6}, P_{4,7}, P_{4,8}, P_{4,9}, ... P_{3,N} corresponding to the reception beam-forming curve CV_{6,3} from the pixels P_{a,b} of the ultrasound image UI, wherein 1≤a≤M, 1≤b≤N. That is, the receiving section 330 selects the pixels P_{3,1}, P_{3,2}, P_{4,2}, P_{4,3}, P_{4,4}, P_{4,5}, P_{4,6}, P_{4,7}, P_{4,8}, P_{4,9}, ... P_{3,N} on which the reception beam-forming curve CV_{6,3} passes among the pixels P_{a,b} of the ultrasound image UI. The receiving section 330 is also configured to assign the sampling data S_{6,3} to the selected pixels P_{3,1}, P_{3,2}, P_{4,2}, P_{4,3}, P_{4,4}, P_{4,5}, P_{4,6}, P_{4,7}, P_{4,8}, P_{4,9}, ... P_{3,N}, as shown in FIG. 6.

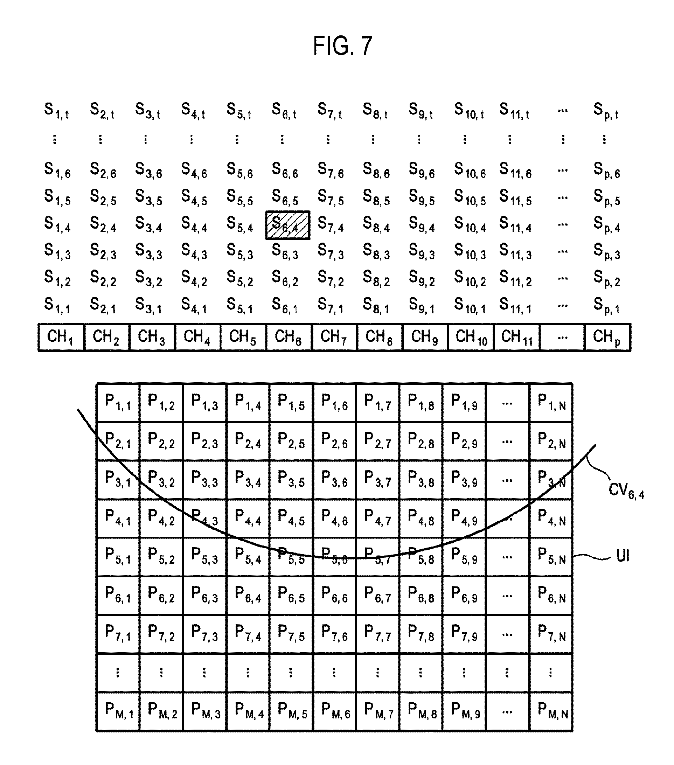

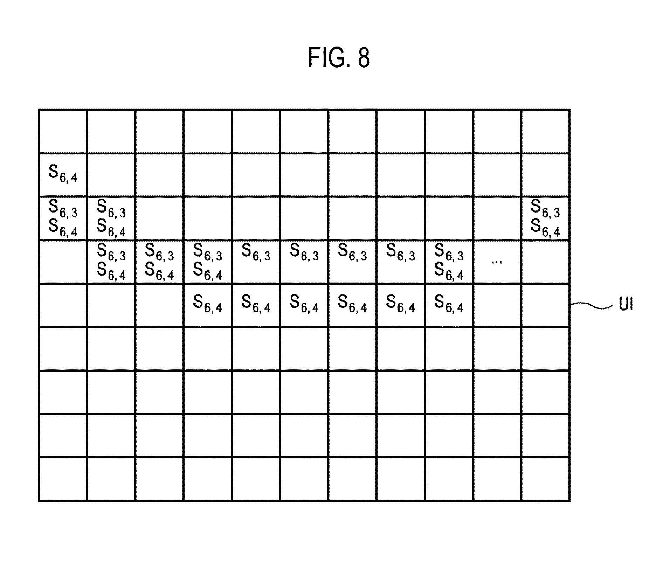

Thereafter, the receiving section 330 is configured to set a reception beam-forming curve CV_{6,4} for selecting pixels, which the sampling data S_{6,4} are used as the pixel data thereof, during the reception beam-forming based on the positions of the elements and the orientation of the respective pixels of the ultrasound image UI with respect to the elements, as shown in FIG. 7. The receiving section 330 is further configured to detect the pixels P_{2,1}, P_{3,1}, P_{3,2}, P_{4,2}, P_{4,3}, P_{4,4}, P_{5,4}, P_{5,5}, P_{5,6}, P_{5,7}, P_{5,8}, P_{4,9}, P_{5,9}, ... P_{4,N}, P_{3,N} corresponding to the reception beam-forming curve CV_{6,4} from the pixels P_{a,b} of the ultrasound image UI. That is, the receiving section 330 selects the pixels P_{2,1}, P_{3,1}, P_{3,2}, P_{4,2}, P_{4,3}, P_{4,4}, P_{5,4}, P_{5,5}, P_{5,6}, P_{5,7}, P_{5,8}, P_{4,9}, P_{5,9}, ... P_{4,N}, P_{3,N} on which the reception beam-forming curve CV_{6,4} passes among the pixels P_{a,b} of the ultrasound image UI. The receiving section 330 is further configured to assign the sampling data S_{6,4} to the selected pixels P_{2,1}, P_{3,1}, P_{3,2}, P_{4,2}, P_{4,3}, P_{4,4}, P_{5,4}, P_{5,5}, P_{5,6}, P_{5,7}, P_{5,8}, P_{5,9}, ... P_{4,N}, P_{3,N}, as shown in FIG. 8. In this way, the respective sampling data, which are used as the pixel data, are cumulatively assigned to the pixels as the pixel data.

The receiving section 330 is configured to perform the reception beam-forming (i.e., summing) upon the sampling data, which are cumulatively assigned to the respective pixels P_{a,b} of the ultrasound image UI to form the reception-focused data.

In another embodiment, the receiving section 330 is configured to perform the analog-digital conversion upon the reception signals provided through the plurality of channels CHₖ from the ultrasound probe 310 to form the sampling data S_{i,j}, as shown in FIG. 4. The sampling data S_{i,j} are stored in the storage unit 140. The receiving section 330 is further configured to detect pixels corresponding to the sampling data based on the positions of the elements and the position (orientation) of the pixels of the ultrasound image UI with respect to the elements. That is, the receiving section 330 selects the pixels, which the respective sampling data are used as the pixel data thereof, during the reception beam-forming based on the positions of the elements and the orientation of the respective pixels of the ultrasound image UI with respect to the elements. The receiving section 330 is configured to cumulatively assign the sampling data corresponding to the selected pixels as the pixel data. The receiving section 330 is further configured to determine pixels existing in the same column among the selected pixels. The receiving section 330 is also configured to set weights corresponding to the respective determined pixels. The receiving section 330 is additionally configured to apply the weights to the sampling data of the respective pixels.

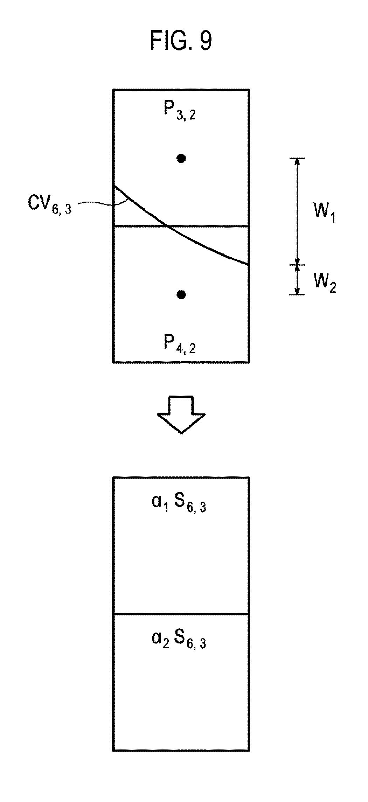

For example, the receiving section 330 is configured to set the reception beam-forming curve CV_{6,3} for selecting pixels, which the sampling data S_{6,3} are used as the pixel data thereof, during the reception beam-forming based on the positions of the elements and the orientation of the respective pixels of the ultrasound image UI with respect to the elements, as shown in FIG. 5. The receiving section 330 is further configured to detect the pixels P_{3,1}, P_{3,2}, P_{4,2}, P_{4,3}, P_{4,4}, P_{4,5}, P_{4,6}, P_{4,7}, P_{4,8}, P_{4,9}, ... P_{3,N} corresponding to the reception beam-forming curve CV_{6,3} from the pixels P_{a,b} of the ultrasound image UI, wherein 1≤a≤M, 1≤b≤N. That is, the receiving section 330 selects the pixels P_{3,1}, P_{3,2}, P_{4,2}, P_{4,3}, P_{4,4}, P_{4,5}, P_{4,6}, P_{4,7}, P_{4,8}, P_{4,9}, ... P_{3,N} on which the reception beam-forming curve CV_{6,3} passes among the pixels P_{a,b} of the ultrasound image UI. The receiving section 330 is also configured to assign the sampling data S_{6,3} to the selected pixels P_{3,1}, P_{3,2}, P_{4,2}, P_{4,3}, P_{4,4}, P_{4,5}, P_{4,6}, P_{4,7}, P_{4,8}, P_{4,9},... P_{3,N}, as shown in FIG. 6. The receiving section 330 is further configured to determine pixels P_{3,2} and P_{4,2}, which exist in the same column among the selected pixels P_{3,1}, P_{3,2}, P_{4,2}, P_{4,3}, P_{4,4}, P_{4,5}, P_{4,6}, P_{4,7}, P_{4,8}, P_{4,9}, ... P_{3,N}. The receiving section 330 is further configured to calculate a distance W₁ from a center of the determined pixel P_{3,2} to the reception beam-forming curve CV_{6,3} and a distance W₂ from a center of the determined pixel P_{4,2} to the reception beam-forming curve CV_{6,3}, as shown in FIG. 9. The receiving section 330 is additionally configured to set a first weight α₁ corresponding to the pixel P_{3,2} based on the distance W₁ and a second weight α₂ corresponding to the pixel P_{4,2} based on the distance W₂. The first weight α₁ and the second weight α₂ are set to be in proportional to or in inverse proportional to the calculated distances. The receiving section 330 is further configured to apply the first weight α₁ to the sampling data S_{6,3} assigned to the pixel P_{3,2} and to apply the second weight α₂ to the sampling data S_{6,3} assigned to the pixel P_{4,2}. The receiving section 330 is configured to perform the above process upon the remaining sampling data.

The receiving section 330 is configured to perform the reception beam-forming upon the sampling data, which are cumulatively assigned to the respective pixels P_{a,b} of the ultrasound image UI to form the reception-focused data.

In yet another embodiment, the receiving section 330 is configured to perform the analog-digital conversion upon the reception signals provided through the plurality of channels CHₖ from the ultrasound probe 310 to form the sampling data S_{i,j}, as shown in FIG. 4. The sampling data S_{i,j} are stored in the storage unit 140. The receiving section 330 is further configured to set a sampling data set based on the sampling data S_{i,j}. That is, the receiving section 330 sets the sampling data set for selecting pixels, which the sampling data S_{i,j} are used as the pixel data thereof, during the reception beam-forming.

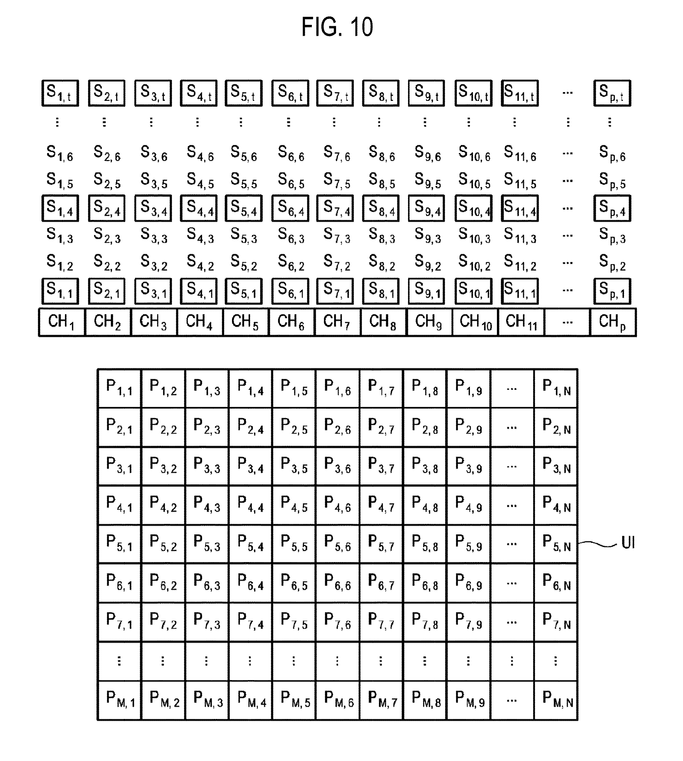

For example, the receiving section 330 is configured to set the sampling data S_{1,1}, S_{1,4}, ... S_{1,t}, S_{2,1}, S_{2,4}, ... S_{2,t}, ... S_{p,t} as the sampling data set (denoted by a box) for selecting the pixels, which the sampling data S_{i,j} are used as the pixel data thereof, during the reception beam-forming, as shown in FIG. 10.

The receiving section 330 is further configured to detect the pixels corresponding to the respective sampling data of the sampling data set based on the positions of the elements and the positions (orientation) of the respective pixels of the ultrasound image UI with respect to the elements. That is, the receiving section 330 selects the pixels, which the respective sampling data of the sampling data set are used as the pixel data thereof, during the reception beam-forming based on the positions of the elements and the orientation of the respective pixels of the ultrasound image UI with respect to the elements. The receiving section 330 is further configured to cumulatively assign the sampling data to the selected pixels in the same manner with the above embodiments. The receiving section 330 is also configured to perform the reception beam-forming upon the sampling data, which are cumulatively assigned to the respective pixels of the ultrasound image UI to form the reception-focused data.

In yet another embodiment, the receiving section 330 is configured to perform a down-sampling upon the reception signals provided through the plurality of channels CHₖ from the ultrasound probe 310 to form down-sampling data. As described above, the receiving section 330 is further configured to detect the pixels corresponding to the respective sampling data, based on the positions of the elements and the positions (orientation) of the respective pixels of the ultrasound image UI with respect to the elements. That is, the receiving section 330 selects the pixels, which the respective sampling data are used as the pixel data thereof, during the reception beam-forming based on the positions of the elements and the orientation of the pixels of the ultrasound image UI with respect to the elements. The receiving section 330 is further configured to cumulatively assign the respective sampling data to the selected pixels in the same manner of the above embodiments. The receiving section 330 is further configured to perform the reception beam-forming upon the sampling data, which are cumulatively assigned to the respective pixels of the ultrasound image UI to form the reception-focused data.

However, it should be noted herein that the reception beam-forming may not be limited thereto.

Referring back to FIG. 3, the ultrasound data acquiring unit 120 further includes an ultrasound data forming section 340. The ultrasound data forming section 340 is configured to form the ultrasound data corresponding to the ultrasound image based on the reception-focused data provided from the receiving section 330. The ultrasound data forming section 340 is further configured to perform a signal process (e.g., gain control, etc) upon the reception-focused data.

In one embodiment, the ultrasound data forming section 340 is configured to form ultrasound data (hereinafter referred to as "brightness mode ultrasound data") corresponding to the brightness mode image BI based on the brightness mode reception-focused data provided from the receiving section 330. The brightness mode ultrasound data include radio frequency data. However, it should be noted herein that the brightness mode ultrasound data may not be limited thereto.

The ultrasound data forming section 340 is further configured to form ultrasound data (hereinafter referred to as "Doppler mode ultrasound data") corresponding to the at least two sample volumes based on the Doppler mode reception-focused data provided from the receiving section 330. The Doppler mode ultrasound data include in-phase/quadrature data. However, it should be noted herein that the Doppler mode ultrasound data may not be limited thereto.

For example, the ultrasound data forming section 340 forms first Doppler mode ultrasound data corresponding to the sample volume SV1 based on the first Doppler mode reception-focused data provided from the receiving section 330. The ultrasound data forming section 340 further forms second Doppler mode ultrasound data corresponding to the sample volume SV₂ based on the second Doppler mode reception-focused data provided from the receiving section 330. The ultrasound data forming section 340 further forms third Doppler mode ultrasound data corresponding to the sample volume SV₃ based on the third Doppler mode reception-focused data provided from the receiving section 330.

Referring back to FIG. 1, the ultrasound system 100 further includes a processing unit 130 in communication with the user input unit 110 and the ultrasound data acquiring unit 120. The processing unit 130 includes a central processing unit, a microprocessor, a graphic processing unit and the like.

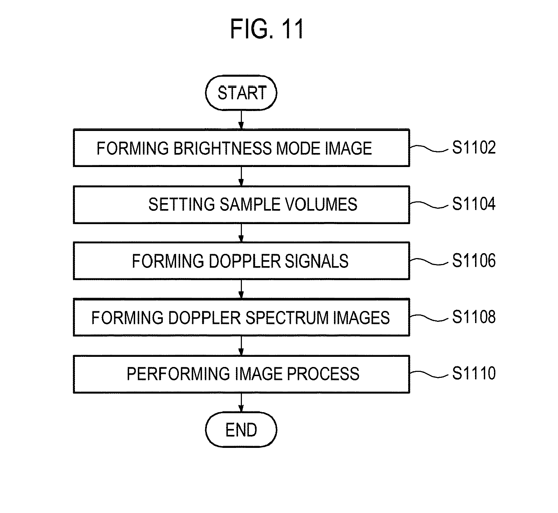

FIG. 11 is a flow chart showing a process of forming the Doppler spectrum images. The processing unit 130 is configured to form the brightness mode image BI based on the brightness mode ultrasound data provided from the ultrasound data acquiring unit 120, at step S1102 in FIG. 11. The brightness mode image BI is displayed on a display unit 150.

The processing unit 130 is configured to set the at least two sample volumes on the brightness mode image BI based on the input information provided from the user input unit 110, at step S1104 in FIG. 11. Thus, the ultrasound data acquiring unit 120 is configured to transmit the ultrasound signals to the living body and receive the ultrasound echo signals from the living body to acquire the Doppler mode ultrasound data corresponding to each of the at least two sample volumes.

The processing unit 130 is configured to form Doppler signals corresponding to each of the at least two sample volumes based on the Doppler mode ultrasound data provided from the ultrasound data acquiring unit 120, at step S1106 in FIG. 11. The methods of forming the Doppler signals are well known in the art. Thus, they have not been described in detail so as not to unnecessarily obscure the present disclosure.

For example, the processing unit 130 forms first Doppler signals corresponding to the sample volume SV₁ based on the first Doppler mode ultrasound data provided from the ultrasound data acquiring unit 120. The processing unit 130 further forms second Doppler signals corresponding to the sample volume SV₂ based on the second Doppler mode ultrasound data provided from the ultrasound data acquiring unit 120. The processing unit 130 further forms third Doppler signals corresponding to the sample volume SV₃ based on the third Doppler mode ultrasound data provided from the ultrasound data acquiring unit 120.

The processing unit 130 is configured to form the at least two Doppler spectrum images corresponding to the at least two sample volumes based on the Doppler signals corresponding to the at least two sample volumes, at step S1108 in FIG. 11. The methods of forming the Doppler spectrum image are well known in the art. Thus, they have not been described in detail so as not to unnecessarily obscure the present disclosure.

For example, the processing unit 130 forms a first Doppler spectrum image corresponding to the sample volume SV₁ based on the first Doppler signals corresponding to the sample volume SV₁. The processing unit 130 further forms a second Doppler spectrum image corresponding to the sample volume SV₂ based on the second Doppler signals corresponding to the sample volume SV₂. The processing unit 130 further forms a third Doppler spectrum image corresponding to the sample volume SV₃ based on the third Doppler signals corresponding to the sample volume SV₃.

The processing unit 130 is configured to perform an image process for connecting the at least two Doppler spectrum images to the at least two sample volumes, at step S1110 in FIG. 11. In one embodiment, the processing unit 130 is configured to set different first connection information on the at least two sample volumes. The processing unit 130 is further configured to set second connection information corresponding to the first connection information on the at least two Doppler spectrum images. The first and second connection information includes at least one of a color, a figure, a numerical value, a text and an image.

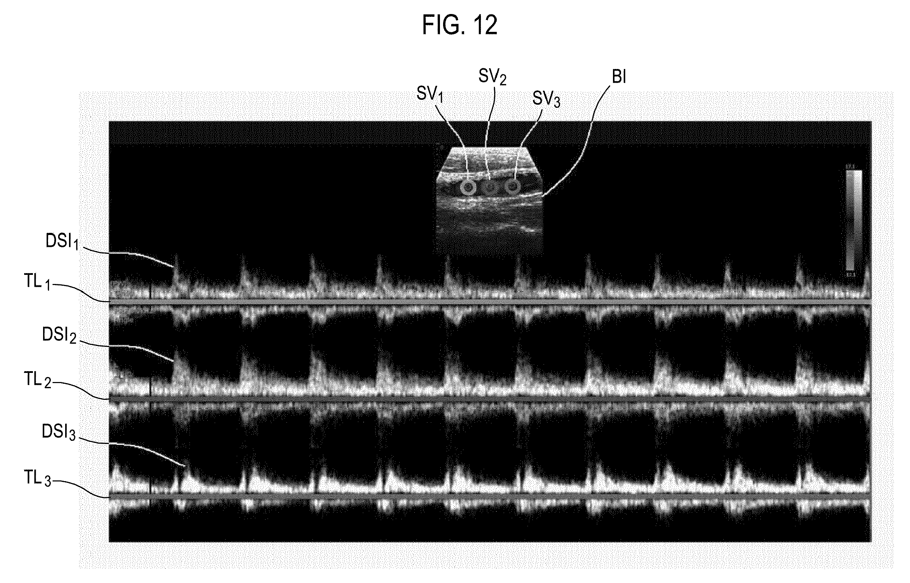

As one example, the processing unit 130 sets the different first connection information (e.g., color) on the sample volumes SV_{1,} SV₂ and SV₃. That is, the processing unit 130 sets yellow on the sample volume SV₁, sets red on the sample volume SV₂, and sets blue on the sample volume SV₃. The processing unit 130 further sets the second connection information (e.g., timeline marker) TL₁, TL₂, TL₃ corresponding to the colors set on the sample volumes SV₁, SV₂, SV₃ on the Doppler mode images DSI₁, DSI₂, DSI₃ corresponding to the sample volumes SV₁, SV₂, SV₃, as shown in FIG. 12. That is, the processing unit 130 sets the timeline marker TL₁ corresponding to the color (i.e., yellow) set on the sample volume SV₁ on the first Doppler spectrum image DSI₁ corresponding to the sample volume SV_{1.} The processing unit 130 further sets the timeline marker TL₂ corresponding to the color (i.e., red) set on the sample volume SV₂ on the second Doppler spectrum image DSI₂ corresponding to the sample volume SV₂. The processing unit 130 further sets the timeline marker TL₃ corresponding to the color (i.e., blue) set on the sample volume SV₃ on the third Doppler spectrum image DSI₃ corresponding to the sample volume SV₃.

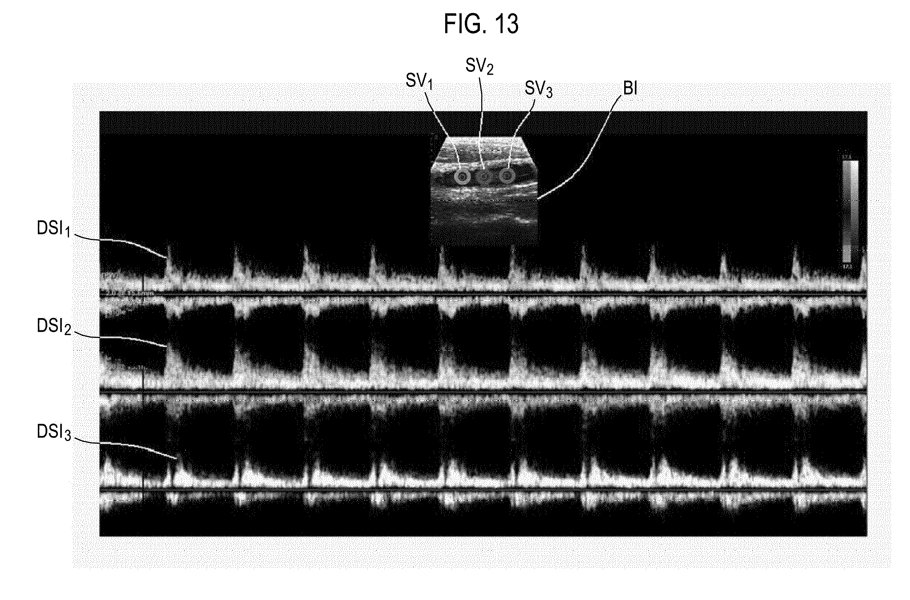

As another example, the processing unit 130 sets the different first connection information (e.g., color) on the sample volumes SV₁, SV₂, SV₃, as shown in FIG. 13. That is, the processing unit 130 sets yellow on the sample volume SV_{1,} sets red on the sample volume SV₂, ands set blue on the sample volume SV₃. The processing unit 130 further performs a color-mapping upon a part or all of the Doppler spectrum images DSI₁, DSI₂, DSI₃ corresponding to the sample volumes SV₁, SV₂, SV₃ with the second connection information corresponding to the colors set on the sample volumes SV₁, SV₂, SV₃ to. That is, the processing unit 130 performs the color-mapping upon a part or all of the first Doppler spectrum image DSI₁ corresponding to the sample volume SV₁ with the second connection information (i.e., yellow) corresponding to the color (i.e., yellow) set on the sample volume SV_{1.} The processing unit 130 further performs the color-mapping upon a part or all of the second Doppler spectrum image DSI₂ corresponding to the sample volume SV₂ with the second connection information (i.e., red) corresponding to the color (i.e., red) set on the sample volume SV₂. The processing unit 130 further performs the color-mapping upon a part or all of the third Doppler spectrum image DSI₃ corresponding to the sample volume SV₃ with the second connection information (i.e., blue) corresponding to the color (i.e., blue) set on the sample volume SV₃.

Referring back to FIG. 1, the ultrasound system 100 further includes the storage unit 140. The storage unit 140 stores the ultrasound data (i.e., brightness mode ultrasound data and Doppler mode ultrasound data) acquired by the ultrasound data acquiring unit 120. The storage unit 140 further stores the Doppler signals formed by the processing unit 130. The storage unit 140 further stores the input information received by the user input unit 110.

The ultrasound system 100 further includes the display unit 150. The display unit 150 is configured to display the brightness mode image formed by the processing unit 130. The display unit 150 is further configured to display the Doppler spectrum images formed by the processing unit 130.

Although embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
a processing unit configured to form at least two Doppler spectrum images corresponding to at least two sample volumes based on ultrasound data corresponding to the at least two sample volume, the processing unit being further configured to perform an image process for connecting the at least two sample volumes to the at least two Doppler spectrum images.

2. The ultrasound system of Claim 1, wherein the processing unit is configured to:
set different first connection information on the at least two sample volumes; and
set second connection information corresponding to the first connection information on the at least two Doppler spectrum images.

3. The ultrasound system of Claim 2, wherein the first connection information includes at least one of a color, a figure, a numerical value, a text and an image, and the second connection information includes at least one of a color, a figure, a numerical value, a text and an image.

4. The ultrasound system of Claim 1, further comprising:
an ultrasound data acquiring unit configured to transmit ultrasound signals to a living body in at least one transmission direction and receive ultrasound echo signals from the living body in at least one reception direction to acquire the ultrasound data corresponding to the at least two sample volumes.

5. The ultrasound system of Claim 4, wherein the ultrasound data acquiring unit is configured to:
form reception signals based on the ultrasound echo signals;
perform an analog-digital conversion upon the reception signals to form a plurality of sampling data;
set a beam-forming curve for selecting pixels which the respective sampling data are used as pixel data thereof; and
select the pixels corresponding to the beam-forming curve to detect pixels corresponding to each of the sampling data from the pixels of the Doppler spectrum images to cumulatively assign the sampling data to the detected pixels;
perform reception beam-forming upon the sampling data assigned to the detected pixels to form reception-focused data corresponding to the at least two sample volumes; and
form the ultrasound data corresponding to the at least two sample volume based on the reception-focused data.

6. The ultrasound system of Claim 5, wherein the ultrasound data acquiring unit is further configured to:
determine pixels existing in the same column of the Doppler spectrum images among the selected pixels;
calculate distances from a center of the respective determined pixels to the beam-forming curve;
set the weights based on the calculated distances; and
apply the weights to the sampling data of the respective determined pixels.

7. The ultrasound system of Claim 5, wherein the ultrasound data acquiring unit is further configured to:
set a sampling data set for selecting pixels which the respective sampling data are used as pixels data thereof among the sampling data; and
select pixels corresponding to respective sampling data of the sampling data set.

8. The ultrasound system of Claim 5, wherein the ultrasound data acquiring unit is further configured to:
perform a down-sampling process upon the reception signals to form down-sampled data.

9. A method of providing Doppler spectrum images, comprising:
a) forming at least two Doppler spectrum images corresponding to at least two sample volumes based on ultrasound data corresponding to the at least two sample volumes; and
b) performing an image process for connecting the at least two sample volumes to the at least two Doppler spectrum images.

10. The method of Claim 9, wherein the step b) comprises:
setting different first connection information on the at least two sample volumes; and
setting second connection information corresponding to the first connection information on the at least two Doppler spectrum images.

11. The method of Claim 10, wherein the first connection information includes at least one of a color, a figure, a numerical value, a text and an image, and the second connection information includes at least one of a color, a figure, a numerical value, a text and an image.

12. The method of Claim 9, further comprising:
transmitting ultrasound signals to a living body including the target object in at least one transmission direction and receiving ultrasound echo signals from the living body in at least one reception direction to acquire the ultrasound data corresponding to the at least two sample volumes, prior to performing the step a).

13. The method of Claim 12, wherein the step of acquiring the ultrasound data, further comprises:
forming reception signals based on the ultrasound echo signals;
performing an analog-digital conversion upon the reception signals to form a plurality of sampling data;
setting a beam-forming curve for selecting pixels which the respective sampling data are used as pixel data thereof; and
selecting the pixels corresponding to the beam-forming curve to detect pixels corresponding to each of the sampling data from the pixels of the Doppler spectrum images to cumulatively assign the sampling data to the detected pixels;
performing reception beam-forming upon the sampling data assigned to the detected pixels to form reception-focused data corresponding to the at least two sample volumes; and
forming the ultrasound data corresponding to the at least two sample volume based on the reception-focused data.
the sampling data to detected pixels;

14. The method of Claim 13, wherein the step of acquiring the ultrasound data, further comprises:
determining pixels existing in the same column of the Doppler spectrum images among the selected pixels;
calculating distances from a center of the respective determined pixels to the beam-forming curve;
setting the weights based on the calculated distances; and
applying the weights to the sampling data of the respective determined pixels.

15. The method of Claim 13, wherein the step of detecting the pixels corresponding to each of the sampling data, further comprises:
setting a sampling data set for selecting pixels, which the respective sampling data are used as pixel data thereof, among the sampling data; and
selecting pixels corresponding to respective sampling data of the sampling data set.

16. The method of Claim 13, wherein the step of detecting the pixels corresponding to each of the sampling data, further comprises:
performing a down-sampling process upon the reception signals to form down-sampled data.
